# EUROPÄISCHE PATENTSCHRIFT

(11) **EP 1 165 153 B1**
(45) Veröffentlichungstag und Bekanntmachung des Hinweises auf die Patenterteilung: **23.03.2005**
(21) Anmeldenummer: 00920662.4
(22) Anmeldetag: 05.04.2000
(51) Int. Cl.: A61L 9/014, C09D 183/04, C08K 3/22

(54) **BESCHICHTUNGSMASSE AUF SILANBASIS MIT KATALYTISCHER OXIDATIVER UND DESODORISIERENDER WIRKUNG**
SILANE-BASED COATING MASS WITH A CATALYTIC, OXIDATIVE AND DEODORIZING EFFECT
MASSE DE REVETEMENT A BASE DE SILANE AVEC AGENTS D'OXYDATION CATALYTIQUES ET EFFET DESODORISANT

(30) Priorität: 06.04.1999 DE 19915377
(43) Veröffentlichungstag der Anmeldung: 02.01.2002
(73) Patentinhaber: INSTITUT FÜR NEUE MATERIALIEN GEM. GMBH, 66123 Saarbrücken (DE)
(72) Erfinder: BENTHIEN, Thomas, D-86899 Landsberg am Lech (DE); FABER, Stefan, D-66687 Wadern (DE); JONSCHKER, Gerhard, D-66583 Spiesen-Elversberg (DE); SEPEUR, Stefan, D-66787 Wadgassen-Schaffhausen (DE); SCHMIDT, Helmut, D-66130 Saarbrücken-Güdingen (DE); STÖSSEL, Philipp, D-66125 Saarbrücken (DE)
(74) Vertreter: Barz, Peter
(86) Internationale Anmeldenummer: PCT/EP2000/003020
(87) Internationale Veröffentlichungsnummer: WO 2000/059554

(56) Entgegenhaltungen:
- EP-A- 0 459 003
- EP-A- 0 604 919
- EP-A- 0 643 014
- EP-A- 0 842 967
- US-A- 5 624 667

## Beschreibung

Die Erfindung betrifft eine katalytische Zusammensetzung, ein Verfahren zu ihrer Herstellung und die Verwendung der katalytischen Zusammensetzung zum Zwecke der Desodorierung und Oxidation von organischen Komponenten oder Kohlenstoff.

EP-A-0604919 beschreibt ein Desodorierungsmittel, das Kompositteilchen umfasst, die eine Ceroxyverbindung und/oder eine Titanoxidverbindung enthalten. Das Desodorierungsmittel eignet sich zur Geruchsverminderung bei übelriechenden Gasen und für die Verwendung in Sanitärgegenständen.

EP-A-0842967 beschreibt einen Verbundwerkstoff, bei dem ein Substrat auf Basis von Glasfasern, Mineralfasern oder Holzwerkstoffen mit einem Nanokomposit in funktionellem Kontakt steht, wobei das Nanokomposit durch Oberflächenmodifizierung von kolloidalen anorganischen Partikeln mit einem oder mehreren Silanen erhalten wird. Als Beispiel für kolloidale anorganische Partikel wird z.B. TiO₂ genannt.

EP-A-0643014 beschreibt ein Desodorierungsmittel, bei dem Aktivkohle mit Metallsalzen imprägniert und thermisch behandelt wird, um mit Metalloxid belegte Aktivkohleteilchen zu erhalten, die katalytisches Oxidationsvermögen aufweisen. Diese werden mit Polymerteilchen gemischt, um schließlich als Formkörper in Kühlschränken desodorierend zu wirken.

US-A-5624667 beschreibt desodorierend wirkende TiO₂-Teilchen mit spezifischen Anteilen an Zink- oder Zink/Siliciumoxiden.

Aufgabe der vorliegenden Erfindung ist die Bereitstellung von katalytischen Zusammensetzungen, die in der Lage sind, Geruchsbelastungen in der Umgebung zu verringern oder zu beseitigen (Desodorierung), und die organische Komponenten oder Kohlenstoff oxidieren können.

Dieses Ziel wird überraschenderweise durch eine katalytische Zusammensetzung erreicht, die eine Beschichtung aus einer Beschichtungsmasse auf einem Träger umfaßt und erhältlich ist durch Aufbringen der Beschichtungsmasse, umfassend (1) ein Polykondensat aus
(A) einem oder mehreren Silanen der allgemeinen Formel (I)

   RₐSiX₍₄₋ₐ₎ (I)

   worin die Reste R gleich oder verschieden sind und nicht hydrolysierbare Gruppen darstellen, die Reste X gleich oder verschieden sind und hydrolysierbare Gruppen oder Hydroxylgruppen bedeuten und a den Wert 0, 1, 2 oder 3 hat, wobei bei mindestens 50 Stoffmengen-% der Silane a größer 0 ist, oder einem davon abgeleiteten Oligomer,
(B) gegebenenfalls einer oder mehreren Verbindungen von glasbildenden Elementen,
und (2) Teilchen von einem oder mehreren katalytisch wirkenden Übergangsmetalloxiden oder Teilchen, die ein oder mehrere katalytisch wirkende Übergangsmetalloxide auf der Oberfläche aufweisen, wobei das Gewichtsverhältnis von Übergangsmetalloxid-Teilchen zu Polykondensat 10 : 1 bis 1 : 10 beträgt, auf den Träger und thermisches Behandeln der aufgebrachten Beschichtungsmasse.

Bei den hydrolysierbaren Silanen (A) sind die hydrolysierbaren Gruppen X beispielsweise Wasserstoff oder Halogen (F, Cl, Br oder I), Alkoxy (vorzugsweise C₁₋₆-Alkoxy, wie z.B. Methoxy, Ethoxy, n-Propoxy, i-Propoxy und Butoxy), Aryloxy (vorzugsweise C₆₋₁₀-Aryloxy, wie z.B. Phenoxy), Acyloxy (vorzugsweise C₁₋₆-Acyloxy, wie z.B. Acetoxy oder Propionyloxy), Alkylcarbonyl (vorzugsweise C₂₋₇-Alkylcarbonyl, wie z.B. Acetyl), Amino, Monoalkylamino oder Dialkylamino mit vorzugsweise 1 bis 12, insbesondere 1 bis 6 Kohlenstoffatomen.

Bei den nicht hydrolysierbaren Resten R kann es sich um nicht hydrolysierbare Reste R¹ oder um eine funktionelle Gruppe tragende Reste R² handeln, wobei R¹ bevorzugt ist.

Der nicht hydrolysierbare Rest R¹ ist beispielsweise Alkyl (vorzugsweise C₁₋₈-Alkyl, wie Methyl, Ethyl, n-Propyl, Isopropyl, n-Butyl, s-Butyl und t-Butyl, Pentyl, Hexyl, Octyl oder Cyclohexyl), Alkenyl (vorzugsweise C₂₋₆-Alkenyl, wie z.B. Vinyl, 1-Propenyl, 2-Propenyl und Butenyl), Alkinyl (vorzugsweise C₂₋₆-Alkinyl, wie z.B. Acetylenyl und Propargyl) und Aryl (vorzugsweise C₆₋₁₀-Aryl, wie z.B. Phenyl und Naphthyl). Die genannten Reste R¹ und X können gegebenenfalls einen oder mehrere übliche Substituenten, wie z.B. Halogen oder Alkoxy, aufweisen.

Spezielle Beispiele für die funktionellen Gruppen des Restes R² sind die Epoxy-, Hydroxy-, Ether-, Amino-, Monoalkylamino-, Dialkylamino-, Amid-, Carboxy-, Vinyl-, Acryloxy-, Methacryloxy-, Cyano-, Halogen-, Aldehyd-, Alkylcarbonyl-, und Phosphorsäuregruppe. Diese funktionellen Gruppen sind über Alkylen-, Alkenylen- oder Arylen-Brückengruppen, die durch Sauerstoff- oder -NH-Gruppen unterbrochen sein können, an das Siliciumatom gebunden. Die genannten Brückengruppen leiten sich z.B. von den oben genannten Alkyl-, Alkenyl- oder Arylresten ab. Die Reste R² enthalten vorzugsweise 1 bis 18, insbesondere 1 bis 8 Kohlenstoffatome.

In einer bevorzugten Ausführungsform handelt es sich bei den Silanen (A) um eine Mischung aus
(A1) mindestens einem hydrolysierbaren Silan der allgemeinen Formel (II)

   SiX₄ (II)

   in der die Reste X gleich oder verschieden sind und hydrolysierbare Gruppen oder Hydroxylgruppen bedeuten, oder einem davon abgeleiteten Oligomer, und
(A2) mindestens einem Organosilan der allgemeinen Formel (III)

   R¹ ₐ₁R² ₐ₂SiX₍₄₋ₐ₁₋ₐ₂₎ (III)

   in der R¹ gleich oder voneinander verschieden sind und eine nicht hydrolysierbare Gruppe bedeuten, R² gleich oder voneinander verschieden sind und einen eine funktionelle Gruppe tragenden Rest bedeuten, X die vorstehende Bedeutung hat und a1 und a2 den Wert 0, 1, 2 oder 3 haben, wobei die Summe (a1+a2) den Wert 1, 2 oder 3 hat, oder einem davon abgeleiteten Oligomer
in einem Stoffmengenverhältnis (A1):(A2) von 5-50 : 50-95.

In der allgemeinen Formel (III) hat a1 vorzugsweise den Wert 1 oder 2, a2 vorzugsweise den Wert 0, 1 oder 2 und die Summe (a1+a2) vorzugsweise den Wert 1 oder 2.

Besonders bevorzugte hydrolysierbare Silane (A) bzw. (A1) sind Tetraalkoxysilane wie Tetraethoxysilan (TEOS). Besonders bevorzugte hydrolysierbare Silane (A) bzw. (A2) sind Alkyltrialkoxysilane, bevorzugt mit C₁-C₈-Alkyl, insbesondere Methyltriethoxysilan, Aryltrialkoxysilane, insbesondere Phenyltriethoxysilan, Dialkyldialkoxysilane, bevorzugt mit C₁-C₈-Alkyl, insbesondere Dimethyldiethoxysilan, und Diaryldialkoxysilane, insbesondere Diphenyldiethoxysilan. Silane mit funktionellen Gruppen (A) bzw. (A2) sind z. B. Epoxysilane wie 3-Glycidyloxypropyl-trimethoxysilan (GPTS) und Aminosilane wie 3-Aminopropyl-triethoxysilan und 3-(Aminoethylamino)-propyl-triethoxysilan (DIAMO).

In der Silankomponente (A) gemäß Formel (I) ist bei mindestens 50 Stoffmengen-% der Silane a größer 0, d.h., mindestens 50 Stoffmengen-% der Silane enthalten mindestens eine nicht hydrolysierbare Gruppe R. Vorzugsweise enthält die Silankomponente (A) 50 bis 95 Stoffmengen-% Silane mit mindestens einer nicht hydrolysierbaren Gruppe R. Bezüglich der Formeln (II) und (III) ist das bevorzugte Stoffmengenverhältnis des hydrolysierbaren Silans (A1) zu dem Organosilan (A2) im Polykondensat 5 bis 50 : 50 bis 95, vorzugsweise 1:1 bis 1:6 und besonders bevorzugt 1:3 bis 1:5. Ein besonderes günstiges Stoffmengenverhältnis ist 1:4.

Die Eventualkomponente (B) stellt glasbildende Elemente dar, die vorzugsweise im Reaktionsmedium löslich oder dispergierbar sind. Verwendbar sind z.B. Verbindungen (Halogenide, Alkoxide, Carboxylate, Chelate, etc.) von Lithium, Natrium, Kalium, Rubidium, Cäsium, Beryllium, Magnesium, Calcium, Strontium, Barium, Bor, Aluminium, Titan, Zirkon, Zinn, Zink oder Vanadium.

Zur Herstellung des Polykondensats (1) werden die Ausgangskomponenten (A) und gegebenenfalls (B) hydrolysiert und kondensiert. Die Hydrolyse und Kondensation wird entweder in Abwesenheit eines Lösungsmittels oder vorzugsweise in einem wäßrigen oder wäßrig/organischen Reaktionsmedium, gegebenenfalls in Gegenwart eines sauren oder basischen Kondensationskatalysators wie HCl, HNO₃ oder NH₃ durchgeführt. Bevorzugt erfolgt die Hydrolyse und Kondensation in Gegenwart einer wäßrigen Säure. Die wäßrigen Säuren werden bevorzugt in einem Konzentrationsbereich von 0,1 N bis 10,0 N eingesetzt. Bevorzugt eingesetzte Säuren sind Salz-, Salpeter-, Phosphor- und Essigsäure.

Daneben können bei der Herstellung des Polykondensats die nachstehend aufgeführten anorganischen Partikel zugegeben werden. Vorzugsweise werden bei der Herstellung nanoskalige anorganische Partikel, insbesondere in Form eines Sols, zugegeben. Beispielsweise können Kieselsole im Sol als hydrolytisch aktive Verbindung wirken. Hierfür eignen sich handelsübliche Kieselsole, wie beispielsweise die Levasile®, Kieselsole der Bayer AG.

Bei Einsatz eines flüssigen Reaktionsmediums sind die Ausgangskomponenten in dem Reaktionsmedium löslich. Als organische Lösungsmittel eignen sich insbesondere mit Wasser mischbare Lösungsmittel, wie z.B. ein- oder mehrwertige aliphatische Alkohole, aber auch aliphatische oder aromatische Kohlenwasserstoffe, z. B. mit 5 bis 20 Kohlenstoffatomen, Ether, Ester, Ketone, Amide und Alkylamide.

Vorzugsweise erfolgt die Hydrolyse und Polykondensation unter den Bedingungen des Sol-Gel-Prozesses, wobei das Reaktionsgemisch im viskosen Sol-Zustand zum Beschichten des Substrats verwendet wird.

Gegebenenfalls wird die Hydrolyse und Polykondensation in Gegenwart eines Komplexbildners durchgeführt, z.B. Nitraten, β-Dicarbonylverbindungen (z.B. Acetylacetonaten oder Acetessigsäurester), Carbonsäuren (z.B. Methacrylsäure) oder Carboxylaten (z.B. Acetat, Citrat oder Glykolat), Betainen, Diolen, Diaminen (z.B. DIAMO) oder Kronenether.

Das Verhältnis der hydrolytisch aktiven Komponenten zu den hydrolysierbaren Silanen (und gegebenenfalls den glasbildenden Elementen) kann durch den R_{OR}-Wert gekennzeichnet werden. Der R_{OR}-Wert stellt das Stoffmengenverhältnis von Wasser aus den hydrolytisch aktiven Komponenten (Wasser, wäßrige Säure, Kieselsol usw.) zu den vorstehend aufgeführten hydrolysierbaren Gruppen X aus den Silankomponenten (und gegebenenfalls den entsprechenden hydrolysierbaren Gruppen der glasbildenden Elementen) dar. Das erhaltene Sol besitzt beispielsweise einen R_{OR}-Wert von 0,1 bis 10 und bevorzugt von 0,2 bis 2.

Das erhaltene Polykondensat wird bevorzugt in Form eines Sols mit Teilchen von einem oder mehreren Übergangsmetalloxiden vermischt, wobei das Verhältnis von Übergangsmetalloxid-Teilchen zu Polykondensat 10 : 1 bis 1 : 10, bevorzugt 10 : 1 bis 1 : 1 und besonders bevorzugt 10 : 1 bis 2 : 1 beträgt. Bei diesem Verhältnis werden für das Polykondensat mit Ausnahme von gegebenenfalls hinzugefügtem organischem Lösungsmittel die zur Herstellung des Polykondensats hinzugegebenen Komponenten (insbesondere die anorganischen Partikel zur Herstellung des Kondensats) berücksichtigt.

Der durchschnittliche Teilchendurchmesser der eingesetzten Übergangsmetalloxide liegt beispielsweise in einem Bereich von 10 nm bis 20 µm. Bei beschichteten Substraten, die zur Geruchsverbesserung eingesetzt werden sollen, werden bevorzugt Übergangsmetalloxid-Teilchen mit einem durchschnittlichen Teilchendurchmesser von 1 bis 20 µm eingesetzt.

Die Teilchen bestehen im wesentlichen beziehungsweise bevorzugt vollständig aus Übergangsmetalloxid. Die Übergangsmetalloxid-Teilchen können aus einem Übergangsmetalloxid oder aus Übergangsmetalloxid-Mischungen bestehen. Bei den bevorzugt eingesetzten Übergangsmetalloxid-Mischungen werden bevorzugt verschiedene Übergangsmetalloxidpulver miteinander vermengt, so daß Teilchen aus verschiedenen Übergangsmetalloxiden vorliegen. Selbstverständlich können auch Teilchen, die verschiedene Übergangsmetalloxide enthalten, eingesetzt werden.

Insbesondere bei der Verwendung für Oxidationszwecke können aber neben den im wesentlichen aus Übergangsmetalloxiden bestehenden Teilchen, auch teilweise oder vollständig Teilchen eingesetzt werden, die an der Oberfläche die nachstehend aufgeführten Übergangsmetalloxide aufweisen, ansonsten aber aus einem anderen Material bestehen. Die Übergangsmetalloxid-Teilchen bestehen dann aus Teilchen eines Materials, das vorzugsweise aus einem der nachstehend für die anorganischen Partikel genannten Materialien gewählt ist, das an der. Oberfläche mit einem oder mehreren Übergangsmetalloxiden beschichtet ist. Vorzugsweise sind diese Teilchen an der Oberfläche vollständig mit den Übergangsmetalloxiden beschichtet. Diese Teilchen werden bei dem Gewichtsverhältnis von Übergangsmetalloxid-Teilchen zu Polykondensat als Ganzes als Übergangsmetalloxid-Teilchen berücksichtigt. Hierbei handelt es sich insbesondere um die nachstehend aufgeführten Partikel im Mikrometerbereich, die an der Oberfläche mit Übergangsmetalloxiden versehen beziehungsweise imprägniert sind.

Bei den Übergangsmetalloxiden handelt es sich um katalytisch wirkende Übergangsmetalloxide, die desodorierende und/oder oxidierende Eigenschaften aufweisen. Unter den Übergangsmetallen werden wie üblich die Elemente der Nebengruppen I bis VIII des Periodensystems und die Lanthaniden- und Actiniden-Elemente verstanden. Das Übergangsmetalloxid wird besonders bevorzugt ausgewählt aus den Oxiden der Metalle La, Ce, Ti, Zr, V, Cr, Mo, W, Mn, Fe, Co, Ni, Cu, Ag und Zn oder aus Mischungen dieser Metalloxide. Bevorzugt eingesetzt werden Übergangsmetalloxid-Mischungen, wobei Mischungen der Oxide von Mn und Ce mit einem oder zwei weiteren Übergangsmetallen, wie Mischungen der Oxide von Mn/Co/Ce, Mn/Cu/Ce, Mn/Ni/Ce, Mn/Fe/Ce oder Mn/Co/Ni/Ce, besonders bevorzugt sind. Eine weiter bevorzugte Übergangsmetalloxid-Mischung ist eine Mischung der Oxide von Cu/V/La. Es können auch Mischoxide der vorgenannten Übergangsmetalle eingesetzt werden.

In den Übergangsmetalloxid-Mischungen sind folgende Mengen der entsprechenden Metalloxide in der Metalloxid-Mischung bevorzugt: Ce: 1-70 Gew.%, V: 5-70 Gew.%, Mn: 20-95 Gew.%, Fe: 20-95 Gew.%, Co: 1-50 Gew.%, Ni: 1-50 Gew.%, Cu: 1-95 Gew.%.

Konkrete Beispiele für Übergangsmetalloxide sind MnO₂ (Pyrolusit), γ-MnO₂, Co₃O₄, Co₂O₃, CoO und CeO₂. Natürlich kann auch jedes andere geeignete Übergangsmetalloxid eingesetzt werden.

Die BET-Oberfläche der eingesetzten Teilchen liegt beispielsweise in einem Bereich von 1 bis 100 m²/g.

Neben den Übergangsmetalloxid-Teilchen können in der Beschichtungsmasse zusätzlich Co-Katalysatoren, z. B. in Mengen von 1 bis 5 Gew.-%, bezogen auf die Übergangsmetalloxid-Teilchen, eingesetzt werden. Geeignete Co-Katalysatoren sind etwa K-, Mg-, Ca-, Ba- und Sr-Salze sowie Al- und Sn-Oxid. Als Salze eignen sich beispielsweise die entsprechenden Halogenide, Hydroxide, Nitrate, Carbonate, Phosphate oder Carbonate. Die Zugabe kann z.B. durch Mischen des Co-Katalysators mit den Übergangsmetalloxid-Teilchen oder mit den Mischungen der Übergangsmetalloxid-Teilchen vor Zugabe zum Polykondensat oder durch separate Zugabe des Co-Katalysators in die Beschichtungsmasse erfolgen. Im ersteren Fall werden bevorzugt Pulver eingesetzt und im letzteren Fall verwendet man bevorzugt leicht lösliche Salze des Co-Katalysators.

In der Beschichtungsmasse können auch anorganische Partikel enthalten sein, die bei der Herstellung des Polykondensats oder der Beschichtungsmasse oder danach zugegeben werden können. Dabei kann es sich um nanoskalige anorganische Partikel oder um Partikel im Mikrometerbereich handeln. Es können auch Partikel beider Größenordnungen zugegeben werden, wobei die Partikel im Mikrometerbereich insbesondere bei Verwendung der katalytischen Zusammensetzung zur Oxidation von organischen Komponenten oder Kohlenstoff eingesetzt werden.

Die anorganischen Partikel können aus beliebigen Materialien bestehen, vorzugsweise handelt es sich um Oxide. Bevorzugte Oxide sind Oxide von Si und Al (insbesondere Boehmit). Die Partikel können z. B. in Form von Pulvern oder, insbesondere die nanoskaligen, in Form von Solen zugegeben werden.

Die nanoskaligen anorganischen Partikel besitzen vorzugsweise eine durchschnittliche Teilchengröße bis zu 300, insbesondere bis zu 100 nm und besonders bevorzugt bis zu 50 nm. Die Partikel können in kolloidaler Form zugegeben werden. Dabei kann es sich um Sole oder dispergierbare Pulver handeln. Spezielle Beispiele für nanoskalige anorganische Partikel sind SiO₂, Al₂O₃, SnO₂, Eisenoxide oder Kohlenstoff (Ruß und Graphit), insbesondere SiO₂. Ganz bevorzugt werden Kieselsole als nanoskalige anorganische Teilchen eingesetzt.

Insbesondere wenn die katalytischen Zusammensetzungen als oxidativ wirkende Zusammensetzungen eingesetzt werden sollen, können zur Beschichtungsmasse auch anorganische Partikel im Mikrometerbereich zugegeben werden. Sie dienen zur Strukturierung der Beschichtung und zur Erzeugung von Hohlräumen. Diese Partikel besitzen einen durchschnittlichen Partikeldurchmesser von beispielsweise 1 bis 500, bevorzugt 10 bis 300 µm. Es handelt sich dabei bevorzugt um oxid- und/oder hydroxylhaltige Verbindungen der Elemente der III und IV Hauptgruppe wie Aluminium- oder Siliciumoxide. Sie können aktiviert sein. Als Beispiele können Kieselgur, Aluminiumoxid 90, Kieselgel 40 oder Kieselgel 60, hergestellt von der Firma Merck, genannt werden.

Die oben genannten anorganischen Partikel im Mikrometerbereich können vor dem Einsatz mit Metallsalzen oder Mischungen von Metallsalzen, z. B. Chloriden, Phosphaten, Formiaten, Nitraten oder Acetaten, getränkt und anschließend bei erhöhten Temperaturen getempert werden, um katalytisch aktive Metalloxide auf der Oberfläche zu erzeugen. Bevorzugt werden Metallnitrate oder Metallacetate eingesetzt, da die Anionen bei der Behandlung im verwendeten Temperaturbereich flüchtige Produkte bilden. Als Metalle werden die für die Übergangsmetalloxid-Teilchen genannten Übergangsmetalle eingesetzt. Man erhält dann Partikel, die an der Oberfläche mit Übergangsmetalloxiden versehen sind, und die erfindungsgemäß als Übergangsmetalloxid-Teilchen eingesetzt werden, und beim Gewichtsverhältnis von Übergangsmetalloxid-Teilchen zu Polykondensat als Ganzes berücksichtigt werden.

Die Beschichtungsmasse kann auch weitere Additive enthalten. Es können z.B. Additive verwendet werden, die sich zur Viskositätseinstellung und/oder insbesondere zur Erzeugung von Hohlräumen bei der thermischen Behandlung der Beschichtungsmassen eignen. Hierfür können z.B. übliche Verdickungsmittel eingesetzt werden. Konkrete Beispiele sind Cellulose-Derivate, z. B. Hydroxypropylcellulose, Stärke, modifizierte Stärke, Polyvinylalkohol und Glycole, z. B. Polyethylenglycol. Bevorzugt werden Cellulose-Derivate, insbesondere Hydroxypropylcellulose verwendet. Daneben können auch die in katalytischen Zusammensetzungen üblichen Additive wie Pigmente (z.B. Schwarzpigmente) eingesetzt werden.

Die Viskosität des mit den Übergangsmetalloxid-Teilchen vermischten Sols kann gegebenenfalls noch durch Entfernen oder Zugabe eines Lösungsmittels, z. B. eines der vorstehend genannten, eingestellt werden. Es ist in dieser Form üblicherweise auch lange lagerfähig. Gegebenenfalls kann es durch Zugabe von Wasser oder wäßriger Säure aktiviert werden, wobei die Beschichtungsmasse dann bevorzugt innerhalb eines Monats eingesetzt wird.

Die Beschichtungsmasse wird nach üblichen Beschichtungsmethoden auf den Träger aufgebracht. Anwendbare Techniken sind z.B. das Tauchen, Gießen, Schleudern, Aufsprühen oder Aufstreichen.

Geeignete Träger sind z.B. solche aus Metallen wie Edelstahl, Stahl, Kupfer, Messing und Aluminium; Metalloxiden, Gläsern wie Floatglas, Borosilikatglas, Bleikristall oder Kieselglas; Glaskeramiken, und Keramiken wie Al₂O₃, ZrO₂, SiO₂-Mischoxiden oder auch Email, aber auch poröse Träger wie z.B. poröse Keramiken. Die Form der Träger ist beliebig. Es kann sich um ebene oder strukturierte Träger handeln. Besonders geeignet sind Träger in Form von Geflechten, Wabenkörpern oder Netzen, etwa Drahtgeflechte, beispielsweise Stahldrahtgeflechte, keramische Wabenkörper oder Drahtnetze.

Vor dem Auftrag der Beschichtungsmasse können die Träger vorbehandelt werden. Beispielsweise werden sie einer Reinigung, z. B. mit handelsüblichen alkalischen Reinigern, unterworfen. Ebenfalls kann z.B. durch das Tempern von Stahlträgern und die Ausbildung von Chromoxidwhiskem an der Oberfläche eine wesentlich verbesserte Haftung des Beschichtungsmaterials auf Stahlträgern erreicht werden.

Der erhaltene Überzug wird gegebenenfalls vorgetrocknet und dann thermisch behandelt. Dies kann bei Temperaturen von 200°C bis 700°C, vorzugsweise 300°C bis 400°C, erfolgen. Die thermische Behandlung kann an der Luft oder in einem Inertgas wie Stickstoff oder Argon durchgeführt werden. Die Wärmebehandlung kann gegebenenfalls auch durch IR- oder Laser-Strahlung erfolgen. Bei der thermischen Behandlung kann es z.B. zur Trocknung, Härtung oder Verfestigung bzw. Verdichtung der Beschichtungsmasse kommen.

Die Beschichtung wird bevorzugt so ausgeführt, daß Schichtdicken von 0,01 bis 500 µm, bevorzugt 1 bis 500 µm, erhalten werden. Werden die katalytischen Zusammensetzungen zum Zwecke der Desodorierung eingesetzt, sind Schichtdicken von 30 bis 100 µm, insbesondere 25 bis 75 µm, bevorzugt. Werden die katalytischen Zusammensetzungen als oxidativ wirksame Oberflächen eingesetzt, sind bei Verwendung von Übergangsmetalloxiden mit einer durchschnittlichen Teilchengröße von unter 200 nm Schichtdicken von 1 bis 10 µm geeignet. Die als oxidativ wirksame Oberflächen dienenden katalytischen Zusammensetzungen, die zusätzlich anorganische Partikel im Mikrometerbereich enthalten, weisen bevorzugt Schichtdicken von 100 bis 400 µm auf.

Die erfindungsgemäßen katalytischen Zusammensetzungen können eine poröse oder eine nicht poröse Beschichtung aufweisen. Vorzugsweise weisen die katalytischen Zusammensetzungen poröse Beschichtungen auf. Bei den Poren kann es sich dabei um mikroskopisch sichtbare Hohlräume an der Oberfläche und/oder um feinere Mikroporen handeln. Die unter dem Mikroskop auf der Oberfläche sichtbaren Hohlräume zeigen etwa eine globuläre Morphologie (Halbkugeln) und ihr Durchmesser beträgt etwa 1 bis 5 µm. Ihre Ausdehnung und Form im Innern der Schicht läßt sich mikroskopisch nicht feststellen. Die Bestimmung der BET-Oberflächen von bevorzugten Ausführungsformen weist darauf hin, daß alternativ oder zusätzlich feinere Mikroporen darin vorhanden sind.

Die erfindungsgemäße katalytische Zusammensetzung wirkt desodorierend, d.h. durch Substanzen verursachte Geruchsbelastungen können verringert oder ganz vermieden werden. Die desodorierende Wirkung wird insbesondere bei Temperaturen über 150°C, z.B. bei Temperaturen von 150 bis 500°C, bevorzugt 200 bis 350°C, festgestellt. Die geruchsbelastete Luft wird dabei an der katalytischen Zusammensetzung bei erhöhten Temperaturen vorbeigeleitet. Dabei werden in der Luft befindliche Substanzen abgebaut.

Die katalytische Zusammensetzung ist auch in der Lage organische Komponenten oder Kohlenstoff, z.B. Ruß oder Graphit, die sich beispielsweiseauf der Oberfläche der katalytischen Zusammensetzung befinden, zu oxidieren. Die oxidierende Wirkung wird insbesondere bei den vorstehend ausgeführten Temperaturbereichen festgestellt.

Aufgrund dieser Eigenschaften wird die katalytische Zusammensetzung bevorzugt dort eingesetzt, wo Geruchsbelastungen auftreten können oder wo besonders "geruchsneutrale" Luft (also Luft mit möglichst wenig zusätzlichen Stoffen) wünschenswert ist bzw. wo die Oxidation von organischen Komponenten oder Kohlenstoff gewünscht ist. Allgemein können die katalytischen Zusammensetzungen z. B. bei der Viehzucht, der Lebensmittelverarbeitung, z. B. Fischverarbeitung oder in Käsereien, in Fabrikationsprozessen, bei der Müllverarbeitung oder allgemein in chemischen Industrieanlagen aber auch in Wohnräumen verwendet werden. Konkrete Beispiele für Anwendungsgebiete sind daher Toiletten aller Art, Bäder, Transportmittel, wie Automobile, Verbrennungsanlagen, insbesondere deren Abgaseinrichtungen, z. B. der (Diesel)auspuff eines Automobils, Wohnwagen, Tanks, Stallungen, Tankstellen, Faultürme von Kläranlagen, Kompostieranlagen, Güllestationen, Silos, Abluftanlagen aller Art, Atemschutzmasken, Kleiderschränke, Windeln, Müllcontainer oder Gassensoren. Die katalytische Zusammensetzungen werden dabei bevorzugt so eingesetzt, daß sie sich direkt auf irgendeiner Oberfläche des jeweiligen Gegenstandes befinden, wobei dann diese Oberfläche als Substrat dient, oder sie befinden sich in einer gegebenenfalls über eine Anschlußleitung verbundenen Zusatzeinrichtung innerhalb oder in der Nähe des Gegenstandes bzw. des Raumes, in der sich die Gegenstände befinden.

### BEISPIELE

### A. Herstellung der Silansole

### Silansol 1: MTKS-Sol, R_{OR} = 0,4

Eine Mischung aus 1069,86 g (6,0 mol) Methyltriethoxysilan und 312,48 g (1,5 mol) Tetraethoxysilan wird in zwei Portionen (Portion 1 und Portion 2) gleichen Gewichts geteilt.
Zu Portion 1 gibt man unter gutem Rühren 246,84 g Kieselsol (Levasil 300/30, wäßrig, basisch, Bayer AG). Nachdem sich eine Emulsion gebildet hat (ca. 30 s) gibt man 5,60 g 36 Gew.-%ige HCl zu. Nach kurzem Rühren (30-50 s) wird die Reaktionsmischung unter Erwärmen klar. Zu dieser Reaktionsmischung gibt man die Portion 2 in einem Zuge schnell zu. Nach kurzer Zeit trübt sich die Reaktionsmischung durch einen farblosen Niederschlag (NaCl). Anschließend rührt man noch 15 min unter Kühlung in einem Eisbad nach. Man läßt das Silanhydrolysat 12 h bei Raumtemperatur stehen, dekantiert vom sedimentierten Feststoff ab, und erhält so das gebrauchsfähige MTKS-Sol.

### Silansol 2: MDKS-Sol, ROR = 0,2

Eine Mischung aus 356,62 g (2,0 mol) Methyltriethoxysilan und 74,14 g (0,5 mol) Dimethyldiethoxysilan wird unter gutem Rühren gleichzeitig mit 35,10 g Kieselsol (Levasil 300/30, wäßrig, basisch, Bayer AG) und 1,10 g 36 Gew.-%iger HCl versetzt. Nach kurzem Rühren (30-50 s) wird die Reaktionsmischung unter Erwärmen klar. Nach kurzer Zeit trübt sich die Reaktionsmischung durch einen farblosen Niederschlag (NaCl). Anschließend rührt man noch 15 min unter Kühlung in einem Eisbad nach. Man läßt das Silanhydrolysat 12 h bei Raumtemperatur stehen, dekantiert vom sedimentierten Feststoff ab, und erhält so das gebrauchsfähige MDKS-Sol.

### Silansol 3: MPTKS-Sol, ROR = 0,4

Eine Mischung aus 11,59 g (0,065 mol) Methyltriethoxysilan, 3,61 g (0,015 mol) Phenyltriethoxysilan und 4,17 g (0,020 mol) Tetraethoxysilan wird unter gutem Rühren gleichzeitig mit 3,29 g Kieselsol (Levasil 300/30, wäßrig, basisch, Bayer AG) und 0,13 g 36 Gew.-%iger HCl versetzt. Nach kurzem Rühren (30-50 s) wird die Reaktionsmischung unter Erwärmen klar. Nach kurzer Zeit trübt sich die Reaktionsmischung durch einen farblosen Niederschlag (NaCl). Anschließend rührt man noch 15 min unter Kühlung in einem Eisbad nach. Man läßt das Silanhydrolysat 12 h bei Raumtemperatur stehen, dekantiert vom sedimentierten Feststoff ab, und erhält so das gebrauchsfähige MPTKS-Sol.

### Silansol 4: MPrTKS-Sol, ROR = 0,4

Eine Mischung aus 15,00 g (0,084 mol) Methyltriethoxysilan, 14,95 g (0,091 mol) *n*-Propyltrimethoxysilan und 8,96 g (0,043 mol) Tetraethoxysilan wird unter gutem Rühren gleichzeitig mit 7,00 g Kieselsol (Levasil 300/30, wäßrig, basisch, Bayer AG) und 0,23 g 32 Gew.-%iger HCl versetzt. Nach kurzem Rühren (30-50 s) wird die Reaktionsmischung unter Erwärmen klar. Nach kurzer Zeit trübt sich die Reaktionsmischung durch einen farblosen Niederschlag (NaCl). Anschließend rührt man noch 15 min unter Kühlung in einem Eisbad nach. Man läßt das Silanhydrolysat 12 h bei Raumtemperatur stehen, dekantiert vom sedimentierten Feststoff ab, und erhält so das gebrauchsfähige MPrTKS-Sol.

### Silansol 5: MD-Sol, ROR = 0,4

Eine Mischung aus 35,66 g (0,2 mol) Methyltriethoxysilan und 7,41 g (0,05 mol) Dimethyldiethoxysilan wird mit 5,04 g 0,1N HCl unter gutem Rühren versetzt. Nach kurzem Rühren (30-50 s) wird die Reaktionsmischung unter Erwärmen klar. Man läßt das Silanhydrolysat 12 h bei Raumtemperatur stehen und erhält so das gebrauchsfähige MD-Sol.

### B. Herstellung der Katalysatormischungen

Als Katalysatormischungen werden Mischungen kommerzieller Übergangsmetalloxid-Pulver der Fa. Ferro oder Aldrich verwendet:
- MnO₂ :: Pulver der Fa. Ferro, überwiegend MnO₂ (Pyrolusit), wenig γ-MnO₂ und wenig MnO₂
- Co_{y}Oₓ :: Pulver der Fa. Ferro, überwiegend Co₃O₄, sehr wenig CoO

### Katalysatormischung 1: Mn/Co/Ce

Die Katalysatormischung 1 wird durch inniges Mischen von 800,00 g MnO₂, 100,00 g Co_{y}Oₓ und 100,00 g CeO₂ hergestellt.

### Katalysatormischung 2: Mn/Co/Ce

Die Katalysatormischung 2 wird durch inniges Mischen von 800,00 g MnO₂, 150,00 g Co_{y}Oₓ und 50,00 g CeO₂ hergestellt.

### Katalysatormischung 3: Mn/Cu/Ce

Die Katalysatormischung 3 wird durch inniges Mischen von 650,00 g MnO₂, 300,00 g Cu₂O und 50,00 g CeO₂ hergestellt.

### Katalysatormischung 4: Mn/Co/Ni/Ce

Die Katalysatormischung 4 wird durch inniges Mischen von 700,00 g MnO₂, 100,00 g Co_{y}Oₓ, 150,00 g NiO und 50,00 g CeO₂ hergestellt.

### C. Herstellung der Beschichtungsmassen

### Beispiel 1

1000,00 g Katalysatormischung 1 werden mit 300,00 g Silansol 1 und 233,33 g Ethanol 2 h bei Raumtemperatur gerührt. Anschließend fügt man zur Aktivierung (Erhöhung des R_{OR}-Werts von 0,4 auf 0,8) 32,35 g 10 Gew.-%ige wäßrige Salzsäure zu, rührt mindestens 2 h bei Raumtemperatur nach, und erhält so die gebrauchsfertige Beschichtungssuspension.

### Beispiel 2

1000,00 g Katalysatormischung 2 werden mit 200,00 g Silansol 1 und 350,00 g Ethanol 2 h bei Raumtemperatur gerührt. Anschließend fügt man zur Aktivierung (Erhöhung des R_{OR}-Werts von 0,4 auf 0,8) 23,49 g 10 Gew.-%ige wäßrige Salzsäure zu, rührt mindestens 2 h bei Raumtemperatur nach, und erhält so die gebrauchsfertige Beschichtungssuspension.

### Beispiel 3

1000,00 g Katalysatormischung 3 werden mit 400,00 g Silansol 2 und 185,00 g Ethanol 1 h bei Raumtemperatur gerührt. Anschließend fügt man zur Aktivierung (Erhöhung des R_{OR}-Werts von 0,2 auf 0,6) 47,97 g 10 Gew.-%ige wäßrige Salzsäure zu, rührt mindestens 4 h bei Raumtemperatur nach, und erhält so die gebrauchsfertige Beschichtungssuspension.

### Beispiel 4

100,00 g Katalysatormischung 3 werden mit 18,00 g Silansol 3 und 25,00 g Ethanol 1 h bei Raumtemperatur gerührt. Anschließend fügt man zur Aktivierung (Erhöhung des R_{OR}-Werts von 0,4 auf 0,7) 1,52 g 10 Gew.-%ige wäßrige Salzsäure zu, rührt mindestens 2 h bei Raumtemperatur nach, und erhält so die gebrauchsfertige Beschichtungssuspension.

### Beispiel 5

100,00 g Katalysatormischung 4 werden mit 40,00 g Silansol 5 und 11,00 g Ethanol 1 h bei Raumtemperatur gerührt. Anschließend fügt man zur Aktivierung (Erhöhung des R_{OR}-Werts von 0,4 auf 0,8) 4,66 g 10 Gew.-%ige wäßrige Salzsäure zu, rührt mindestens 2 h bei Raumtemperatur nach, und erhält so die gebrauchsfertige Beschichtungssuspension.

### D. Beschichtung und thermische Behandlung (insbesondere für Desodorierungszwecke)

Als Trägermaterial werden Stahldrahtgeflechte (Durchmesser ca. 5 cm, Höhe ca. 1 cm) oder keramische Wabenkörper verwendet. Die Stahlgeflechte werden zunächst mit einem kommerziellen, alkalischen Reiniger entfettet, anschließend mit deionisiertem Wasser gut nachgespült, und dann bei Raumtemperatur getrocknet. Die trockenen Stahlgeflechte werden anschließend 1h bei 500°C getempert.

Die Beschichtung erfolgt durch Tränken der Stahldrahtgeflechte oder der keramischen Wabenkörper in einer der unter Abschnitt C beschriebenen Beschichtungsmassen (Beschichtungssuspensionen). Die überschüssige Beschichtungssuspension wird durch Durchblasen mit Preßluft entfernt. Nach Trocknung bei Raumtemperatur (2 h) erfolgt die Verfestigung der Beschichtung durch thermische Behandlung. Die beschichteten Träger wurden dazu innerhalb von 1 h von Raumtemperatur auf 300°-400°C aufgeheizt, 1 h Stunde bei 300°-400°C gehalten, und anschließend während 6 h auf Raumtemperatur abgekühlt.

Alternativ kann die thermische Behandlung auch durch direktes Einstellen der getrockneten, beschichteten Träger in einen auf 300°-400°C vortemperierten Ofen, und schnelles Abkühlen der heißen Träger auf Raumtemperatur während einiger Minuten erfolgen.

Die Schichtdicken der thermisch verfestigten Schichten liegen typischerweise im Bereich von 25-75 µm. Die Schichtdicken können z.B. zum einen durch die Viskosität der Beschichtungssuspension (diese kann z.B. durch Zugabe einer geeigneten Menge an Ethanol eingestellt werden), zum anderen durch den Druck der Preßluft, bzw. die Einwirkzeit der Preßluft beim Entfernen der überschüssigen Beschichtungssuspension, eingestellt werden.

### E. Katalytische Zusammensetzung 1 (insbesondere für die Oxidation)

### E.1 Darstellung eines Mn/Cu/Ce - Katalysators auf Aluminiumoxid-Partikeln

40,47 g (0,141 mol) Mn(NO₃)₂ · 6 H₂O , 11,63 g (0,050 mol) Cu(NO₃)₂ · 3 H₂O und 15,20 g (0,035 mol) Ce(NO₃)₃ · 6 H₂O werden in einer Mischung aus 30,00 g Ethanol und 30,00 g Wasser bei 50°C gelöst. Zu dieser Lösung gibt man 100,00 g Aluminiumoxid 90 (aktiv, sauer (alternativ kann auch neutrales oder basisches eingesetzt werden), Partikelgröße 63-200 µm, Fa. Merck) und dampft das Lösungsmittelgemisch unter Rühren bei 50-70°C während 3 h ab. Das mit den Metallnitraten imprägnierte Aluminiumoxid wird anschließend 1h bei 500°C getempert. Analog können auch die entsprechenden molaren Mengen an Metallacetaten eingesetzt oder anstelle von Aluminium 90 die ebenfalls von der Fa. Merck hergestellten Kieselgel 40, Partikelgröße 63-200 µm, Kieselgel 60, Partikelgröße 63-200 µm oder Kieselgur, Partikelgröße ca. 100 µm, verwendet werden.

### E.2 Beschichtungsmasse

150,00 g des vorstehend beschriebenen Mn/Cu/Ce-Katalysators (E.1) auf den Aluminiumoxid-Partikeln und 50,00 g Katalysatormischung 1 werden innig gemischt. Zu dieser Mischung fügt man unter Rühren 100,00 g einer 5 Gew.-%igen Lösung von Hydroxypropylcellulose in Ethanol. 140,00 g Silansol 2 werden zur Aktivierung (Erhöhung des R_{OR}- Werts von 0,2 auf 0,8) unter Rühren mit 22,67 g (1,26 mol) Wasser versetzt und 30 min bei Raumtemperatur gerührt. Das aktivierte MDKS-Sol wird unter Rühren bei Raumtemperatur zur oben beschriebenen Mischung aus Mn/Cu/Ce-Katalysator, Katalysatormischung 1 und Hydroxypropylcellulose in Ethanol gefügt und anschließend 15 min bei Raumtemperatur gerührt, um die gebrauchsfertige Beschichtungsmasse zu erhalten.

### E.3 Beschichtung und thermische Verfestigung

Als Trägermaterial werden Stahlsubstrate (Bleche 10 x 10 cm) verwendet. Die Stahlsubstrate werden zunächst mit einem kommerziellen, alkalischen Reiniger entfettet, anschließend mit deionisiertem Wasser gut nachgespült, und dann bei Raumtemperatur getrocknet. Die trockenen Stahlsubstrate können anschließend 1h bei 500°C getempert werden.

Die gereinigten oder die gereinigten und getemperten Stahlsubstrate werden mit der Beschichtungsmasse geflutet. Die beschichteten Stahlsubstrate werden 1 h bei Raumtemperatur getrocknet, anschließend innerhalb von 1 h von Raumtemperatur auf 500°C aufgeheizt, 1 h bei 500°C gehalten, und anschließend innerhalb von 6 h auf Raumtemperatur abgekühlt.

Die Schichtdicken der thermisch verfestigten Schichten liegen typischerweise im Bereich von 150-400 µm, je nach verwendetem Trägermaterial und verwendeter Menge an Beschichtungsmasse.

### F. Katalytische Zusammensetzung 2 (insbesondere für die Oxidation)

### F.1 Herstellung eines Mischoxidkatalysators durch Co-Präzipitation von Mn/Co/Ce

121,42 g (0,423 mol) Mn(NO₃)₂ · 6 H₂O, 14,55 g (0,050 mol) Co(NO₃)₂ · 6 H₂O und 9,42 g (0,022 mol) Ce(NO₃)₃ · 6 H₂O werden in 350,00 g Wasser bei 80°-90°C gelöst. Als Fällungsreagenz wird eine Lösung von 66,77 g (1,19 mol) KOH in 300,00 g Wasser, die unter Rühren mit 1,60 g Tween 80 (Polyoxyethylen(20)-sorbitanmonooleat, Fa. Aldrich) und 1,60 g (0,012 mol) 1-Octanol versetzt wird, verwendet. Die oben beschriebene Lösung der Metallnitrate wird bei 80°-90°C unter Rühren während 5 min mit dem oben beschriebenen Fällungsreagenz versetzt. Die so erhaltene homogene, lehmfarbene Suspension wird weitere 2 h bei 90°C gerührt, anschließend filtriert man vom Niederschlag ab, wäscht diesen zwei mal mit je 150 g Wasser und einmal mit 50 ml Ethanol. Der Niederschlag wird 1 h bei 70°C vorgetrocknet und anschließend innerhalb von 1 h von Raumtemperatur auf 600°C aufgeheizt, 2 h bei 600°C gehalten, und dann innerhalb von 2 h auf Raumtemperatur abgekühlt.

Das so erhaltene, grünliche Pulver wird in 250,00 g Wasser bei 90°C aufgeschlämmt, und dann, nach Zusatz von 1,60 g Tween 80 und 1,60 g (0,012 mol) 1-Octanol, mit Hilfe eines Ultraschalldesintegrators innerhalb von 30 min redispergiert. Anschließend filtriert man vom Niederschlag ab, wäscht diesen zwei mal mit je 50 g Wasser und einmal mit 100 g Ethanol. Der Niederschlag wird 1 h bei 70°C vorgetrocknet und anschießend innerhalb von 1 h von Raumtemperatur auf 250°C aufgeheizt, 1 h bei 250°C gehalten, und dann während 2 h auf Raumtemperatur abgekühlt. Man erhält ein feines, grünliches Katalysatorpulver.

### F.2 Beschichtungsmasse

3,00 g Silansol 2 werden zur Aktivierung (Erhöhung des R_{OR} Werts von 0,2 auf 0,8) unter Rühren mit 0,48 g (0,027 mol) Wasser versetzt und 30 min bei Raumtemperatur gerührt. Zum aktivierten MDKS-Sol gibt man unter Rühren 10,00 g des unter Punkt F.1 beschriebenen Katalysatorpulvers und 8,00 g Ethanol, man rührt 30 min bei Raumtemperatur nach, und erhält so die gebrauchsfähige Beschichtungsmasse.

### F.3 Beschichtung und thermische Verfestigung

Als Trägermaterial werden Stahlsubstrate (Bleche 10 x 10 cm) verwendet. Die Stahlsubstrate werden zunächst mit einem kommerziellen, alkalischen Reiniger entfettet, anschließend mit deionisiertem Wasser gut nachgespült, und dann bei Raumtemperatur getrocknet. Die trockenen Stahlgeflechte können anschließend 1h bei 500°C getempert werden. Die gereinigten oder die gereinigten und getemperten Stahlsubstrate werden mit der Beschichtungsmasse geflutet. Die beschichteten Stahlsubstrate werden 1 h bei Raumtemperatur getrocknet, anschließend innerhalb von 1 h von Raumtemperatur auf 300°-600°C aufgeheizt, 1 h bei 300°-600°C gehalten, und anschließend während 6 h auf Raumtemperatur abgekühlt.

Die Schichtdicken der thermisch verfestigten Schichten liegen typischerweise im Bereich von 1-10 µm, je nach verwendeter Menge an Beschichtungsmasse.

### G. Bewertung

### Verfahren zur Bestimmung der desodorierenden Wirkung

In einen auf 300°C vorgewärmten Umluftofen (Temperatur am Katalysator ca. 300°C, Träger: Stahldrahtgeflechte) werden nacheinander ca. 100 mg der Testsubstanzen:
Pyrazin, Thiazol, Maltol, Vanillin und 2,4-Decadienal eingebracht.

Die Testsubstanzen verdampfen im heißen Umluftofen, die Dämpfe werden über den Umluftstrom als Abgase (Abgasstrom: 0.5-1.2 l/s) durch einen Auslaßstutzen ohne Katalysator und einen Auslaßstutzen mit Katalysator zu einem nachgeschalteten Probensammler geleitet. Die gesammelten Proben werden mit Hilfe der GC-MS-Spektroskopie analysiert. Aus den Spektren werden Abbauraten für die Testsubstanzen im Abgasstrom der über den Katalysator läuft, im Vergleich zum Abgasstrom der nicht über einen Katalysator läuft, ermittelt (Prinzip: Relativmessung an einem experimentellen Aufbau). Die Abbauraten sind nachfolgend in % angegeben.

| Katalysator | Pyrazin | Thiazol | Maltol | Vanilin | Decadienal |
|---|---|---|---|---|---|
| Pd/Pt-Kat(*1) | 0 | 0 | 90 | 90 | -- |
| (*2) | 83 | 88 | 73 | 78 | 65 |
| (*3) | 69 | 56 | 74 | 70 | -- |

| | | | | | |
|---|---|---|---|---|---|
| (*1): Palladium, metallisch, auf Stahldrahtnetzen, handelsüblicher Katalysator. | | | | | |
| (*2): Erfindungsgemäßer Mn/Co/Ce-MTKS-Sol-Kat, Beschichtungsmasse Beispiel 1 | | | | | |
| (*3): Erfindungsgemäßer Mn/Cu/Ce-MDKS-Sol-Kat, Beschichtungsmasse Beispiel 3. | | | | | |

Es zeigt sich, daß die erfindungsgemäßen katalytischen Zusammensetzungen in der Lage sind, neben den anderen Testsubstanzen, auch Heterocyclen wie Pyrazin und Thiazol abzubauen. Dies ist mit handelsüblichen Palladium-Katalysatoren nicht möglich. Außerdem tritt bei den erfindungsgemäßen katalytischen Zusammensetzungen nach 10 Testzyklen kein Verlust an katalytischer Aktivität auf. Dagegen wird der handelsübliche PalladiumKatalysator durch Heterocyclen wie Thiazol vergiftet, d.h. er verliert mit der Zeit an katalytischer Aktivität.

### Bewertung des Oxidationsvermögens

### [Prüfmethode nach DIN 51 171, "Prüfung des Selbstreinigungsvermögens kontinuierlich selbstreinigender Emaillierungen"]

Auf die zu untersuchenden Proben werden an fünf auf einem Kreis angeordneten Stellen so lange definierte Mengen (je 20-25 mg) Soja- oder Motorenöl aufgetropft, und nach jedem Auftropfen durch eine einstündige Wärmebehandlung bei 250°C verbrannt, bis infolge der Ansammlung unverbrannter Rückstände ein sichtbarer Glanz auftritt. Zur Beurteilung dienen die Anzahlen der Zyklen bis zum Glanzauftritt.

| Beschichtung | Öl | Zahl der Zyklen bis Glanz |
|---|---|---|
| (*1) | Soja | 4-5 |
| (*2) | Soja | 15-17 |
| (*3) | Soja | 13-15 |
| (*4) | Motoren | 10-12 |

| | | |
|---|---|---|
| (*1): Handelsübliches, oxidativ wirkendes Email, enthält Fe/Mn-Oxide | | |
| (*2): Katalytische Zusammensetzung 1 | | |
| (*3): Katalytische Zusammensetzung 1, aber unter Verwendung von Kieselgel 40 als Trägermaterial anstelle von Aluminiumoxid 90 | | |
| (*4): Katalytische Zusammensetzung 2, z.B. für den Feuersteg von Motorenkolben | | |

Die erfindungsgemäßen katalytischen Zusammensetzungen (Schichtdicken zwischen 150-400 Mikrometer) weisen aufgrund der vorhandenen Hohlräume in der Beschichtung eine hohe Saugfähigkeit und damit ein gutes Spreitungsvermögen für Öle auf. Im Gegensatz dazu weisen die glasartigen Emails ein geringes Saug- und Spreitungsvermögen auf.

## Patentansprüche

1. Katalytische Zusammensetzung für Desodorisierungs- oder Oxidationszwecke, die eine Beschichtung aus einer Beschichtungsmasse auf einem Träger umfasst und erhältlich ist durch Aufbringen der Beschichtungsmasse, umfassend (1) ein Polykondensat aus
(A) einem oder mehreren Silanen der allgemeinen Formel (I)
RₐSiX₍₄₋ₐ₎ (I)
worin die Reste R gleich oder verschieden sind und nicht hydrolysierbare Gruppen darstellen, die Reste X gleich oder verschieden sind und hydrolysierbare Gruppen oder Hydroxylgruppen bedeuten und a den Wert 0, 1, 2 oder 3 hat, wobei bei mindestens 50 Stoffmengen-% der Silane a größer 0 ist, oder einem davon abgeleiteten Oligomer,
(B) gegebenenfalls einer oder mehreren Verbindungen von glasbildenden Elementen,
und (2) Teilchen von einem oder mehreren katalytisch wirkenden Übergangsmetalloxiden oder Teilchen, die ein oder mehrere katalytisch wirkende Übergangsmetalloxide auf der Oberfläche aufweisen, wobei das Gewichtsverhältnis von Übergangsmetalloxid-Teilchen zu Polykondensat 10 : 1 bis 1 : 10 beträgt, auf den Träger und thermisches Behandeln der aufgebrachten Beschichtungsmasse.

2. Katalytische Zusammensetzung nach Anspruch 1, **dadurch gekennzeichnet, dass** bei 50 bis 95 Stoffmengen-% der Silane a größer 0 ist.

3. Katalytische Zusammensetzung nach Anspruch 1 oder 2, **dadurch gekennzeichnet, dass** das Übergangsmetalloxid ausgewählt ist aus den Oxiden der Metalle La, Ce, Ti, Zr, V, Cr, Mo, W, Mn, Fe, Co, Ni, Cu, Ag und Zn oder Mischungen davon.

4. Katalytische Zusammensetzung nach einem der Ansprüche 1 bis 3 **dadurch gekennzeichnet, dass** der Teilchendurchmesser der Übergangsmetalloxid-Teilchen 10 nm bis 20 µm beträgt.

5. Katalytische Zusammensetzung nach einem der Ansprüche 1 bis 4, **dadurch gekennzeichnet, dass** die Schichtdicke der Beschichtung 0,01 bis 500 µm beträgt.

6. Katalytische Zusammensetzung nach einem der Ansprüche 1 bis 5, **dadurch gekennzeichnet, dass** der Träger aus Metall, Metalloxid, Glas, Glaskeramik, Keramik oder porösem Material besteht.

7. Katalytische Zusammensetzung nach einem der Ansprüche 1 bis 6, **dadurch gekennzeichnet, dass** die Beschichtungsmasse, gegebenenfalls nach vorherigem Trocknen, in einem Temperaturbereich von 200 bis 700°C behandelt worden ist.

8. Katalytische Zusammensetzung nach einem der Ansprüche 1 bis 7, **dadurch gekennzeichnet, dass** in der Beschichtungsmasse zusätzlich anorganische Partikel enthalten sind.

9. Katalytische Zusammensetzung nach einem der Ansprüche 1 bis 8, **dadurch gekennzeichnet, dass** die Beschichtung aus der Beschichtungsmasse porös ist.

10. Katalytische Zusammensetzung nach einem der Ansprüche 1 bis 9, **dadurch gekennzeichnet, dass** die katalytisch wirkenden Übergangsmetalloxid-Teilchen oder die zusätzlichen anorganischen Partikel einen durchschnittlichen Teilchendurchmesser von mindestens 1 µm aufweisen.

11. Verfahren zur Herstellung einer katalytischen Zusammensetzung für Desodorisierungs- oder Oxidationszwecke, die eine Beschichtung aus einer Beschichtungsmasse auf einem Träger umfasst, bei dem (1) ein Polykondensat aus
(A) einem oder mehreren Silanen der allgemeinen Formel (I)
RₐSiX₍₄₋ₐ₎ (I)
worin die Reste R gleich oder verschieden sind und nicht hydrolysierbare Gruppen darstellen, die Reste X gleich oder verschieden sind und hydrolysierbare Gruppen oder Hydroxylgruppen bedeuten und a den Wert 0, 1, 2 oder 3 hat, wobei bei mindestens 50 Stoffmengen-% der Silane a größer 0 ist, oder einem davon abgeleiteten Oligomer,
(B) gegebenenfalls einer oder mehreren Verbindungen von glasbildenden Elementen,
mit (2) Teilchen von einem oder mehreren katalytisch wirkenden Übergangsmetalloxiden oder Teilchen, die ein oder mehrere katalytisch wirkende Übergangsmetalloxide auf der Oberfläche aufweisen, in einem Gewichtsverhältnis von Übergangsmetalloxid-Teilchen zu Polykondensat von 10 : 1 bis 1 : 10 vermischt werden, eine diese Mischung umfassende Beschichtungsmasse auf den Träger aufgetragen wird und gegebenenfalls nach Trocknen thermisch behandelt wird.

12. Verwendung der katalytischen Zusammensetzung nach einem der Ansprüche 1 bis 10 zur Desodorierung.

13. Verwendung der katalytischen Zusammensetzung nach einem der Ansprüche 1 bis 10 zur Oxidation von organischen Komponenten oder Kohlenstoff.

## Claims

1. Catalytic composition for deodorizing or oxidizing purposes which comprises a coating of a coating material on a support and is obtainable by applying the coating material, comprising (1) a polycondensate of
(A) one or more silanes of the general formula (I)
Rₐ-Si-X₍₄₋ₐ₎ (I)
in which the radicals R are identical or different and are non-hydrolysable groups, the radicals X are identical or different and are hydrolysable groups or hydroxyl groups and a has the value 0, 1, 2 or 3, with a being greater than 0 for at least 50 mol% of the silanes, or an oligomer derived therefrom,
(B) if desired, one or more compounds of glass-forming elements,
and (2) particles of one or more catalytically active transition metal oxides or particles having one or more catalytically active transition metal oxides at their surface, the weight ratio of transition metal oxide particles to polycondensate being from 10:1 to 1:10, to the support and subjecting the applied coating material to thermal treatment.

2. Catalytic composition according to Claim 1, **characterized in that** a is greater than 0 for from 50 to 95 mol% of the silanes.

3. Catalytic composition according to Claim 1 or 2, **characterized in that** the transition metal oxide is selected from the oxides of the metals La, Ce, Ti, Zr, V, Cr, Mo, W, Mn, Fe, Co, Ni, Cu, Ag and Zn or mixtures thereof.

4. Catalytic composition according to one of Claims 1 to 3, **characterized in that** the particle diameter of the transition metal oxide particles is from 10 nm to 20 µm.

5. Catalytic composition according to one of Claims 1 to 4, **characterized in that** the thickness of the coating is from 0.01 to 500 µm.

6. Catalytic composition according to one of Claims 1 to 5, **characterized in that** the support is composed of metal, metal oxide, glass, glass ceramic, ceramic or porous material.

7. Catalytic composition according to one of Claims 1 to 6, **characterized in that** the coating material, immediately or after drying, has been treated at an air temperature range from 200 to 700°C.

8. Catalytic composition according to one of Claims 1 to 7, **characterized in that** the coating material additionally includes inorganic particles.

9. Catalytic composition according to one of Claims 1 to 8, **characterized in that** the coating formed from the coating material is porous.

10. Catalytic composition according to one of Claims 1 to 9, **characterized in that** the catalytically active transition metal oxide particles or the additional inorganic particles have an average particle diameter of at least 1 µm.

11. Process for preparing a catalytic composition for deodorizing or oxidizing purposes which comprises a coating of a coating material on a support, wherein (1) a polycondensate of
(A) one or more silanes of the general formula (I)
Rₐ-Si-X₍₄₋ₐ₎ (I)
in which the radicals R are identical or different and are non-hydrolysable groups, the radicals X are identical or different and are hydrolysable groups or hydroxyl groups and a has the value 0, 1, 2 or 3, with a being greater than 0 for at least 50 mol% of the silanes, or an oligomer derived therefrom, and
(B) if desired, one or more compounds of glass-forming elements
is mixed with (2) particles of one or more catalytically active transition metal oxides or particles having one or more catalytically active transition metal oxides at their surface in a weight ratio of transition metal oxide particles to polycondensate of from 10:1 to 1:10, a coating material comprising this mixture is applied to the support and, optionally after drying, is subjected to heat treatment.

12. Use of the catalytic composition according to one of Claims 1 to 10 for deodorizing.

13. Use of the catalytic composition according to one of Claims 1 to 10 for oxidizing organic components or carbon.

## Revendications

1. Composition catalytique pour la désodorisation ou l'oxydation, qui comprend un revêtement constitué d'une masse de revêtement appliquée sur un support et que l'on peut obtenir en appliquant sur le support la masse de revêtement comprenant (1) un polycondensat constitué de :
(A) un ou plusieurs silanes de formule générale (I)
RₐSiX₍₄₋ₐ₎ (I)
dans laquelle les restes R sont identiques ou différents et représentent des groupes non-hydrolysables, les restes X sont identiques ou différents et représentent des groupe hydrolysables ou des groupes hydroxyle, et a a la valeur 0, 1, 2 ou 3, a étant supérieur à 0 pour au moins 50 % de la quantité des silanes, ou un oligomère dérivé dudit ou desdits silanes, et
(B) éventuellement un ou plusieurs dérivés d'éléments vitrifiables,
et (2) des particules d'un ou plusieurs oxydes de métal de transition à action catalytique, ou des particules présentant sur leur surface un ou plusieurs oxydes de métal de transition à action catalytique, le rapport en poids des particules d'oxyde de métal de transition au polycondensat étant de 10 :1 à 1 :10,
et en soumettant la masse de revêtement appliquée à un traitement thermique.

2. Composition catalytique selon la revendication 1, **caractérisée en ce que** a est supérieur à 0 pour 50 à 95 % de la quantité des silanes.

3. Composition catalytique selon la revendication 1 ou 2, **caractérisée en ce que** l'oxyde de métal de transition est choisi parmi les oxydes des métaux La, Ce, Ti, Zr, V, Cr, Mo, W, Mn, Fe, Co, Ni, Cu, Ag et Zn, ou leurs mélanges.

4. Composition catalytique selon l'une quelconque des revendications 1 à 3, **caractérisée en ce que** le diamètre de particule des particules d'oxyde de métal de transition est de 10 nm à 20 µm.

5. Composition catalytique selon l'une quelconque des revendications 1 à 4, **caractérisée en ce que** l'épaisseur de la couche de revêtement est de 0,01 à 500 µm.

6. Composition catalytique selon l'une quelconque des revendications 1 à 5, **caractérisée en ce que** le support est constitué d'un métal, d'un oxyde métallique, de verre, de vitrocéramique, de céramique ou d'un matériau poreux.

7. Composition catalytique selon l'une quelconque des revendications 1 à 6, **caractérisée en ce que** la masse de revêtement a été traitée à une température située dans le domaine allant de 200 à 700 °C, éventuellement après un séchage préalable.

8. Composition catalytique selon l'une quelconque des revendications 1 à 7, **caractérisée en ce que** la masse de revêtement contient en plus des particules minérales.

9. Composition catalytique selon l'une quelconque des revendications 1 à 8, **caractérisée en ce que** le revêtement obtenu à partir de la masse de revêtement est poreux.

10. Composition catalytique selon l'une quelconque des revendications 1 à 9, **caractérisée en ce que** les particules d'oxyde de métal de transition à action catalytique ou les particules minérales supplémentaires ont un diamètre moyen de particule d'au moins 1 µm.

11. Procédé de préparation d'une composition catalytique pour la désodorisation ou l'oxydation, qui comprend un revêtement constitué d'une masse de revêtement appliquée sur un support, procédé dans lequel on mélange selon un rapport en poids des particules d'oxyde de métal de transition au polycondensat de 10 :1 à 1 :10, (1) un polycondensat constitué de :
(A) un ou plusieurs silanes de formule générale (I)
RₐSiX₍₄₋ₐ₎ (I)
dans laquelle les restes R sont identiques ou différents et représentent des groupes non-hydrolysables, les restes X sont identiques ou différents et représentent des groupes hydrolysables ou des groupes hydroxyle, et a a la valeur 0, 1, 2 ou 3, a étant supérieur à 0 pour au moins 50 % de la quantité des silanes, ou un oligomère dérivé dudit ou desdits silanes, et
(B) éventuellement un ou plusieurs dérivés d'éléments vitrifiables,
avec (2) des particules d'un ou plusieurs oxydes de métal de transition à action catalytique ou des particules présentant sur leur surface un ou plusieurs oxydes de métal de transition à action catalytique, on applique sur le support une masse de revêtement comprenant ce mélange et on la soumet à un traitement thermique, éventuellement après séchage.

12. Utilisation de la composition catalytique selon l'une quelconque des revendications 1 à 10 pour la désodorisation.

13. Utilisation de la composition catalytique selon l'une quelconque des revendications 1 à 10 pour l'oxydation de constituants organiques ou du carbone.
